# FASCICULE DE BREVET EUROPEEN

(11) **EP 1 140 981 B1**
(45) Date de publication et mention de la délivrance du brevet: **22.09.2004**
(21) Numéro de dépôt: 00900616.4
(22) Date de dépôt: 19.01.2000
(51) Int. Cl.: C07K 5/06, A61K 38/04, G01N 33/68

(54) **COMPOSES TRIPEPTIDIQUES UTILES A TITRE D'INHIBITEURS SELECTIFS DE L'AMINOPEPTIDASE A ET COMPOSITIONS PHARMACEUTIQUES CORRESPONDANTES**
TRIPEPTIDISCHE VERBINDUNGEN MIT WIRKSAMKEIT ALS SELEKTIVE INHIBITOREN DER AMINOPEPTIDASE A UND SIE ENTHALTENDE PHARMAZEUTISCHE ZUSAMMENSETZUNGEN
TRIPEPTIDE COMPOUNDS USEFUL AS SELECTIVE INHIBITORS OF AMINOPEPTIDASE A AND CORRESPONDING PHARMACEUTICAL COMPOSITIONS

(30) Priorité: 20.01.1999 FR 9900587
(43) Date de publication de la demande: 10.10.2001
(73) Titulaire: INSTITUT NATIONAL DE LA SANTE ET DE LA RECHERCHE MEDICALE (INSERM), 75013 Paris (FR); CENTRE NATIONAL DE LA RECHERCHE SCIENTIFIQUE (CNRS), 75016 Paris (FR)
(72) Inventeur: ROQUES, Bernard, F-75014 Paris (FR); FOURNIE-ZALUSKI, Marie-Claude, F-75011 Paris (FR); BISCHOFF, Laurent, F-75014 Paris (FR); DAVID-BASEI, Christelle, F-92000 Nanterre (FR); LLORENS-CORTES, Catherine, F-91440 Bures sur Yvette (FR)
(74) Mandataire: Le Coupanec, Pascale
(86) Numéro de dépôt international: PCT/FR2000/000112
(87) Numéro de publication internationale: WO 2000/043414

(56) Documents cités:
- EP-A- 0 181 644
- WO-A-99/24461
- E M GORDON ET AL.: "Design of novel inhibitors of aminopeptidases." JOURNAL OF MEDICINAL CHEMISTRY., vol. 31, no. 11, novembre 1988 (1988-11), pages 2199-2211, XP002072921 AMERICAN CHEMICAL SOCIETY. WASHINGTON., US ISSN: 0022-2623
- L BISCHOFF ET AL.: "Characterization and inhibition of aminopeptidase A" PEPTIDES 1996. PROCEEDINGS OF THE 24TH EUROPEAN PEPTIDE SYMPOSIUM, SEPTEMBER 8-13, EDIMBURGH, SCOTLAND,1998, pages 263-264, XP002117802 London
- L MARTIN ET AL.: "Beta-amino thiols inhibit the zinc metallopeptidase activity of tetanus toxin light chain" JOURNAL OF MEDICINAL CHEMISTRY., vol. 41, no. 18, 27 août 1998 (1998-08-27), pages 3450-3460, XP002117803 AMERICAN CHEMICAL SOCIETY. WASHINGTON., US ISSN: 0022-2623
- CHEMICAL ABSTRACTS, vol. 128, no. 20, 18 mai 1998 (1998-05-18) Columbus, Ohio, US; abstract no. 241119, C DAVID ET AL.: "Characterization and inhibition of aminopeptidase A by alpha-mercapto-beta amino acyl dipeptides" XP002117804 & LETT. PEPT. SCI., vol. 4, no. 4-5-6, 1997, pages 411-414,
- CHEMICAL ABSTRACTS, vol. 131, no. 17, 25 octobre 1999 (1999-10-25) Columbus, Ohio, US; abstract no. 223620, L BISCHOFF ET AL.: "Aminopeptidase A: selective inhibition and physiological involvement in CCK8 metabolism" XP002135905 & PEPT. PROC. AM. PEPT. SYMP., 15TH ,1997, pages 674-675, Kluwer, Dordrecht, NL
- L BISCHOFF ET AL.: "Side-chain modified sulfonic analogues of aspartic and glutamic acids: synthesis, proteiction, and incorporation into peptides" JOURNAL OF ORGANIC CHEMISTRY, vol. 64, no. 4, 19 février 1999 (1999-02-19), pages 1420-1423, XP002135903 AMERICAN CHEMICAL SOCIETY. EASTON., US ISSN: 0022-3263
- C DAVID ET AL.: "Investigation of subsite preferences in aminopeptidase A (EC 3.4.11.7) led to the design of the first highly potent and selective inhibitors of this enzyme" JOURNAL OF MEDICINAL CHEMISTRY., vol. 42, no. 25, 23 novembre 1999 (1999-11-23), pages 5197-5211, XP002135904 AMERICAN CHEMICAL SOCIETY. WASHINGTON., US ISSN: 0022-2623

## Description

La présente invention a pour objet de nouveaux composés à intérêts thérapeutiques utiles notamment à titre d'inhibiteurs sélectifs de l'aminopeptidase A et les compositions pharmaceutiques correspondantes.

L'aminopeptidase A est une ectopeptidase à zinc impliquée de façon très spécifique dans la dégradation de substrats peptidiques possédant en position N-terminale un résidu Asp ou Glu.

Parmi les peptides biologiquement actifs du système nerveux central ou périphérique, l'octapeptide C-terminal de la cholécystokinine CCK₈ et l'angiotensine II constituent précisément des substrats physiologiques de l'aminopeptidase A.

La CCK₈ interagit avec deux types de récepteurs, les sites CCK-A principalement situés à la périphérie et les récepteurs CCK-B préférentiellement situés dans le système nerveux central. Au niveau périphérique, la CCK₈ stimule les contractions de la vésicule biliaire et de l'iléon, augmente la mobilité intestinale et stomacale, favorise la sécrétion d'enzymes pancréatiques. Dans le système nerveux central, la CCK₈ régule le comportement alimentaire, favorise la libération d'hormones hypophysaires et induit des modifications comportementales liées à l'anxiété.

En conséquence, l'emploi d'inhibiteurs de la dégradation de la CCK₈ constitue un moyen efficace pour intervenir au niveau de ces différents processus physiogiques et donc les moduler.

En ce qui concerne l'angiotensine II et son métabolite, l'angiotensine III, elles font partie de la cascade angiotensinergique, interagissent avec les récepteurs ATI et ATII et interviennent de façon différente dans le contrôle central et périphérique de la pression sanguine et la libération d'hormones hypophysaires comme la vasopressine.

En conséquence, dans ce cas particulier, il est envisageable de moduler la pression artérielle et la libération de vasopressine en inhibant la dégradation de l'angiotensine II en angiotensine III. Ceci constituerait une voie thérapeutique pour traiter l'hypertension artérielle essentielle et secondaire, l'insuffisance cardiaque et rénale, les troubles de l'homéostasie hydrodynamique, l'infarctus du myocarde et la protéinurie chez les diabétiques.

Des inhibiteurs d'APA ont déjà été proposés dans la littérature. Ils répondent plus particulièrement à la formule générale A suivante :

H₂N-CH(R_{A})CH₂SH A

dans laquelle R_{A} représente préférentiellement une chaine aliphatique substituée par un groupe chargé négativement (Wilk *et al*. (1990) *Neuropeptides* 16, 163-168 ; Chauvel *et al*. (1994) *J. Med*. *Chem*. 37, 1339-1346, *J. Med*. *Chem.* 37, 2950-2956, Bischoff et al. (1999) Proc. 15th Am. Pept. Symp., 674-675; David et al. (1997), Lett. Pept. Sci. 4(4-5-6), 411-414).

Toutefois cette famille de composés présente l'inconvénient majeur d'être dénuée d'un caractère sélectif à l'égard de l'aminopeptidase A.

Parallèlement à l'inhibition de l'aminopeptidase A, ces composés inhibitent également l'aminopeptidase N. C'est ainsi que leurs Ki sont au mieux de l'ordre de 10⁻⁷ M sur l'APA et leur facteur de sélectivité vis à vis de l'APN relativement faible (inférieur à 100).

Il est clair que ce comportement non sélectif constitue un handicap sur le plan thérapeutique.

La présente invention a précisément pour objet de proposer une nouvelle famille de composés qui avantageusement possède un caractère de sélectivité à l'égard de l'APA.

Plus précisément, la présente invention a pour objet un composé de formule générale I : 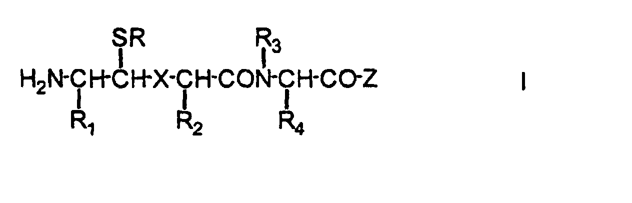 dans laquelle :
- R₁ représente une chaîne alkyle, alkényle ou alkynyle, ou un groupement cycloalkyle, ou (cycloalkyle)alkyle substituée par au moins un groupement
   ― -COOH, éventuellement estérifié par un groupe alkyle comportant de 2 à 12 atomes de carbone,
   ― SO₃H, éventuellement protégé par un groupement pentylique,
   ― PO₃H₂, éventuellement substitué par un groupement (-CH₂CH₂SCOR₅), avec R₅ représentant un groupement alkyle en C₁-C₄, un groupement phényle ou benzyle, ou
   ― tétrazolyle.
- R₂ représente une chaîne alkyle, ou un groupement aryle, arylalkyle, cycloalkyle, (cycloalkyle)alkyle, (héteroaryl)alkyle substituée ou non par au moins un groupement OH, OR, SR', NH₂, NHR', guanidinyle, COOH, CONH₂, ou un atome d'halogène choisi parmi le F, Cl, Br ou I avec R' représentant un alkyle en C₁ à C₄ linéaire ou ramifié.
- R₃ représente un atome d'hydrogène ou un groupement méthyle,
- R₄ représente
   ― une chaîne alkyle, un groupement aryle, arylalkyle, cycloalkyle, (cycloalkyl)alkyle, (hétéroaryl)alkyle, hétérocycloalkyle ou (hétérocycloalkyl)alkyle substituée par au moins un groupement CONH₂, SO₃H, SO₂NH₂, PO₃H₂ ou tétrazolyle, avec le groupement SO₃H pouvant être protégé par un groupement pentylique, et le groupement PO₃H₂ pouvant être protégé par un groupement (-CH₂CH₂SCOR₅) avec R₅ représentant un groupement alkyle en C₁-C₄, un groupement phényle ou benzyle,
   ― un groupement aryle, arylalkyle, cycloalkyle, (cycloalkyl)alkyle, (hétéroaryl)alkyle, hétérocycloalkyle ou (hétérocycloalkyl)alkyle substituée par au moins un groupement CO₂H pouvant être protégé comme décrit ci-dessus, à savoir éventuellement estérifié par un groupe alkyle comportant de 2 à 12 atomes de carbone, ou
- R₃ et R₄ peuvent constituer ensemble un composé hétérocyclique, à 5 ou 6 chaînons, substitué par au moins un groupement CO₂H, CONH₂, SO₃H, SO₂NH₂ ou PO₃H₂ avec les groupements CO₂H, SO₃H et PO₃H₂ le cas échéant protégés comme décrit ci-dessus,
- X représente un groupement CONH ou CH₂NH,
- Z représente un groupement OH, OCH₂-C₆H₅ ou NR"R''' dans lequel R" et R''' peuvent représenter indépendamment l'un de l'autre un atome d'hydrogène ou un groupement alkyle, aryle ou arylalkyle, avec R" et R"' pouvant constituer ensemble, avec l'atome d'azote, un hétérocycle à 5 ou 6 chaînons possédant le cas échéant un 2^{ème} hétéroatome choisi parmi O, S et N,
- R représente un atome d'hydrogène ou un groupement de formule II 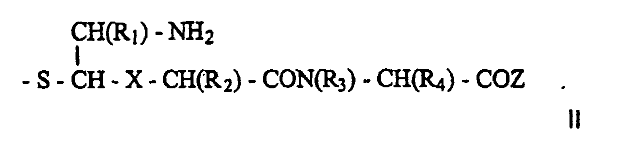 correspondant au disulfure symétrique de l'inhibiteur avec R₁, R₂, R₃, R₄, X et Z étant tels que définis ci-dessus,
   et leurs dérivés.

Les définitions des différents groupements proposés dans la formule générale I des composés revendiqués sont précisées dans la liste ci-dessous. Ces définitions s'appliquent aux termes tels qu'ils sont utilisés à travers ce texte (à moins qu'elles soient limitées à des exemples précis) soit individuellement soit en tant que faisant partie d'un groupe plus large.

C'est ainsi qu'au sens de l'invention, on entend désigner, à défaut de précision contraire, par :
― Alkyle, une chaîne aliphatique saturée linéaire ou ramifiée de 1 à 6 carbones.
― Alkényle, une chaîne aliphatique linéaire ou ramifiée de 2 à 6 carbones possédant une double liaison.
― Alkynyle, une chaîne aliphatique de 2 à 6 carbones possédant une triple liaison.
― Cycloalkyle, un cycle hydrocarboné saturé de 3 à 6 carbones.
― Aryle, un cycle aromatique à 6 carbones, condensé ou non et/ou substitué ou non par un ou deux autres cycles aromatiques à 6 carbones.
― Hétéroaryle, un hétérocycle aromatique à 5 ou 6 chaînons contenant 1 ou 2 hétéroatomes choisi parmi N, S, O, éventuellement condensés ou substitués par un cycle aromatique à 6 carbones.
― Hétérocycloalkyle, un hétérocycle saturé à 5 ou 6 chaînons contenant 1 ou 2 hétéroatomes pris parmi N, S, O éventuellement condensé ou substitué par un cycle saturé à 6 maillons.

Au sens de la présente invention, on entend couvrir sous le terme "dérivés" notamment les sels d'addition des composés de formule générale (I) obtenus avec des acides organiques ou minéraux pharmacologiquement acceptables. Il peut par exemple s'agir de sels tels que les chlorhydrate, bromhydrate, sulfate, nitrate, borate, phosphate, méthane sulfonate, acétate, fumarate, succinate, ascorbate, oxalate, lactate, pyruvate, citrate, tartrate, maléate, malonate, benzoate, diaminobenzène sulfonate, chromoglycate, benzène sulfonate, cyclohexane sulfonate, toluène sulfonate, dipropyl acétate, glucose-1 phosphate, palmoate et palmitate.

Parmi ces dérivés, on peut également citer les dimères de composés de formule générale I, constitués par deux molécules de composés de formule générale I, identiques ou différentes, couplées entre elles au niveau de leurs atomes de soufre respectifs. Dans ce cas particulier, R représente le groupement de formule II identifié précédemment

De même, la présente invention s'étend aux différentes formes éniantomériques des composés revendiqués.

En effet, les composés de formule (I), possédant plusieurs carbones asymétriques, ils existent sous forme de mélanges soit racémiques ou de diastéréoisomères ou encore sous forme de stéréoisomères purs.

Les composés optiquement purs peuvent être isolés par des synthèses énantiosélectives ou des résolutions par des amines chirales. Dans le cas de procédés de préparation conduisant à des mélanges de stéréoisomères, une séparation par HPLC semi-préparative sur colonne (Kromasil C₁₈, 20x250 mm, CH₃CN-H₂O) est effectuée, permettant une étude biochimique et pharmacologique séparée de chaque stéréoisomère.

Parmi ces stéréoisomères, sont préférés ceux possédant une configuration absolue (S) ou (R) sur le carbone portant le groupement R₁, (S) ou (R) sur le carbone portant la fonction -SR, et (S) sur les carbones portant les groupements R₂ et R₄.

Selon un mode préféré de l'invention, les composés revendiqués répondent à la formule générale II dans laquelle
- R₄ représente une chaîne alkyle, un groupement aryle, arylalkyle, cycloalkyle, (cycloalkyl)alkyle, (hétéroaryl)alkyle, hétérocycloalkyle ou (hétérocycloalkyl)alkyle substituée par au moins un groupement CONH₂, SO₃H, SO₂NH₂, PO₃H₂ ou tétrazolyle, avec les groupements SO₃H et PO₃H₂ pouvant être protégés, ou
- R₄ constitue avec R₃ un composé hétérocyclique, à 5 ou 6 chaînons, substitué par au moins un groupement CO₂H, CONH₂, SO₃H, SO₂NH₂ ou PO₃H₂ avec les groupements CO₂H, SO₃H et PO₃H₂ pouvant être protégés comme décrit ci-dessus.

Sont notamment tout particulièrement intéressants les composés revendiqués dans lesquels R₄ et R₃ constituent ensemble un composé hétérocyclique, à 5 ou 6 chaînons, substitué par au moins un groupement CO₂H, CONH₂, SO₃H, SO₂NH₂ ou PO₃H₂ avec les groupements CO₂H, SO₃H et PO₃H₂ pouvant être protégés comme décrit ci-dessus.

Selon un mode préféré de l'invention, les composés revendiqués répondent à la formule générale II dans laquelle X représente une fonction CONH et plus préférentiellement R, un atome d'hydrogène.

Plus préférentiellement, R₂ représente une chaîne alkyle ou arylalkyle substituée le cas échéant et de préférence par un groupement hydroxyle.

A titre illustratif des composés convenant à la présente invention on peut plus particulièrement citer les suivants :
les N-[[(2S,3R) et (2R,3R), 3-amino, 2-sulfhydryl, 5-sulfonate] pentanoyl]-L.Tyr-L.Sal-OH ;
les N-[[(2S,3R) et (2R,3R), 3-amino, 2-sulfhydryl, 5-sulfonate] pentanoyl]-L.Tyr-L.hSal-OH ;
les N-[[(2S,3R) et (2R,3R), 3-amino, 5-carboxy, 2-sulfhydryl] pentanoyl]-L.Ile-L.(3R)(3-COOH)Pro-OH ;
les N-[[(2S,3R) et (2R,3R), 3-amino, 5-phosphonate, 2-sulfhydryl] pentanoyl]-L.Ile-L.Glu-OH ;
les N-[[2S,3R) et (2R,3R), 3-amino, 2-sulfhydryl, 5-sulfonate] pentanoyl]-L.Ile-L.Sal-OH ;
les N-[[(2S,3R) et (2R,3R), 3-amino, 2-sulfhydryl, 5-sulfonate] pentanoyl]-L.Ile-L.(3R)(3-COOH)Pro-OH ;
les N-[[(2S,3R) et (2R,3R), 3-amino, 2-sulfhydryl, 5-sulfonate] pentanoyl]-L.Ile-L.(3S)(3-COOH)Pro-OH ; et
les N-[[(2S,3R) et (2R,3R), 3-amino, 2-sulfhydryl, 5-sulfonate] pentanoyl]-L.Ile-L.Glu-NH₂.

Dans les composés précédents, les abréviations Sal et hSal désignent respectivement le motif sulfoalanine et homosulfoalanine.

Les composés revendiqués sont accessibles par des voies de synthèse différentes selon la définition du groupement X.

La présente invention a également pour objet un procédé utile pour préparer les composés de formule générale (I) dans laquelle X représente un groupement CONH comprenant au moins le couplage d'un dipeptide ester de formule générale III 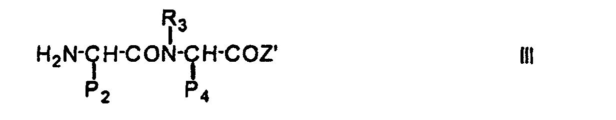 dans laquelle
― P₂ et P₄ correspondent à des formes protégées de R₂ et R₄,
― R₃ est tel que défini ci-dessus et
― Z' représente un groupement OC(CH₃)₃, OCH₂-C₆H₅ ou NR"R"' dans lequel R" et R"' peuvent représenter indépendamment !'un de l'autre un atome d'hydrogène ou un groupement alkyle, aryle ou arylalkyle, avec R" et R''' pouvant constituer ensemble, avec l'atome d'azote, un hétérocycle à 5 ou 6 chaînons possédant le cas échéant un 2^{ème} hétéroatome choisi parmi O, S et N,
avec un composé de formule générale IV 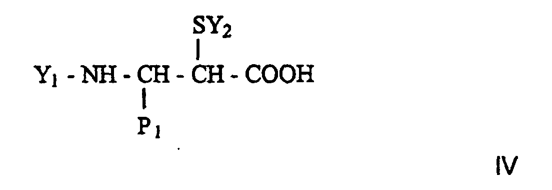 dans laquelle :
― Y₁ représente un groupement protecteur
― Y₂ représente un groupement protecteur et
― P₁ représente une forme protégée de R₁,
dans des conditions suffisantes pour conduire via le composé V 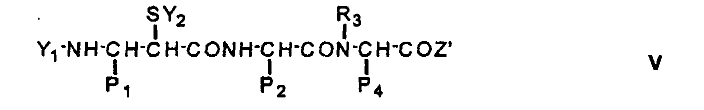 et sa déprotection audit composé de formule générale I.

La réaction de couplage est réalisée de manière conventionnelle dans un solvant organique, en présence d'un agent de couplage et d'une amine tertiaire, à une température de l'ordre de 20°C.

Généralement, le couplage de l'acide de formule (IV) sur l'amine générale (III) est effectué en opérant dans un solvant organique comme le dichlorométhane ou le diméthylformamide, en utilisant comme réactif de couplage, un composé du type BOP [benzotriazol-1-yl-oxy-tris(diméthylamino)phosphonium hexafluorophosphate], HATU [O-(7-azabenzotriazol-1-yl)1,1,3,3-tétraméthyluronium hexafluorophosphate], ou TFFH [tétraméthylfluoro formamidinium hexafluorophosphate] à une température de mélange réactionnel voisine de 20°C et en présence d'une amine tertiaire comme la diisopropyléthylamine.

Les conditions de couplage mettent en jeu préférentiellement comme réactif le BOP (benzotriazol-1-yloxytris(diméthylamino)phosphonium hexafluorophosphate) en présence de diisopropyléthylamine dans le CH₂Cl₂.

Les composés de formule générale (I) dans laquelle X représente un groupement CH₂NH peuvent être obtenus par un procédé comprenant au moins la condensation d'un composé de formule générale III telle que définie ci-dessus et d'un composé de formule générale VI, 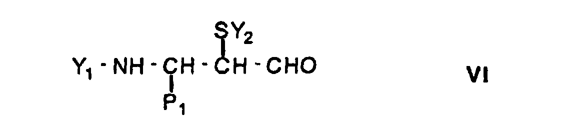 dans laquelle Y₁, Y₂ et P₁ sont tels que définis précédemment puis réduction de l'intermédiaire ainsi formé pour conduire via le composé de formule générale VII 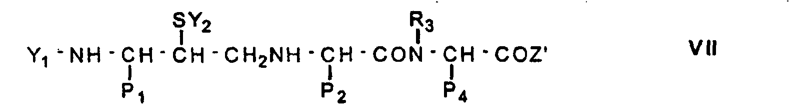 et sa déprotection audit composé de formule générale I.

Le dipeptide ester de formule générale III possède deux carbones asymétriques. Selon un mode préféré de l'invention, on privilégie la configuration S de chaque acide aminé.

En ce qui concerne le composé de formule générale IV, il possède deux carbones asymétriques C₂ et C₃. La stéréochimie de C₃ est définie par celle de l'acide aminé utilisé à l'origine de la synthèse dudit composé IV selon l'un des deux schémas de synthèse définis ci-après.

La réduction de l'intermédiaire peut être réalisée par toute méthode conventionnelle. Généralement, elle est effectuée in situ par ajout d'une quantité suffisante d'un réducteur organique comme un borohydrure et plus préférentiellement du borohydrure de sodium ou du cyanoborohydrure.

En ce qui concerne les réactions de protection et déprotection elles relèvent des compétences de l'homme de l'art.

A titre représentatif des groupements protecteurs susceptibles d'être retenus dans le cadre de la présente invention, on peut notamment privilégier pour Y₁, un groupement t. butyloxycarbonyl (t. Boc), benzyloxycarbonyl (Cbz) ou fluorenyloxycarbonyl (Fmoc), et pour Y₂, un groupement p. methoxybenzyle ou diméthoxybenzyle.

Bien entendu, le choix de ces groupements protecteurs est opéré en considérant la nature de la réaction à faire subir aux composés correspondants et de manière à protéger efficacement, lors du déroulement de cette réaction, les groupes fonctionnels considérés.

Les réactions de déprotection sont bien entendu sélectionnées en fonction de la nature des groupes protecteurs.

Ainsi les composés V et VII avec de préférence Y₁, Y₂, P₁, P₂ et P₄ tels que définis ci-dessus sont généralement déprotégés par traitement avec un mélange TFA/anisole ou BBr₃ dans CH₂Cl₂ ou HBr dans l'acide acétique, ou HF pour conduire aux composés I (avec X = CONH et X = CH₂NH, respectivement).

Toutefois, lorsque P₁ et/ou P₄ contiennent un groupement sulfonate protégé SO₃CH₂t.Bu, une étape supplémentaire de déprotection par chauffage avec du chlorure de tétraméthylammonium dans le DMF est nécessaire pour conduire aux composés I ayant des groupements R₁ et/ou R₃ possédant un sulfonate libre, sauf dans le cas d'une déprotection par HF.

En ce qui concerne les composés de formule générale IV 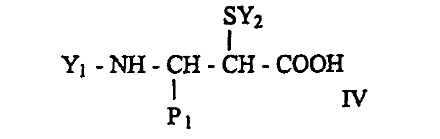 ils sont eux-mêmes accessibles par deux méthodes de synthèse distinctes.

La première voie de synthèse est illustrée par le schéma réactionnel suivant.

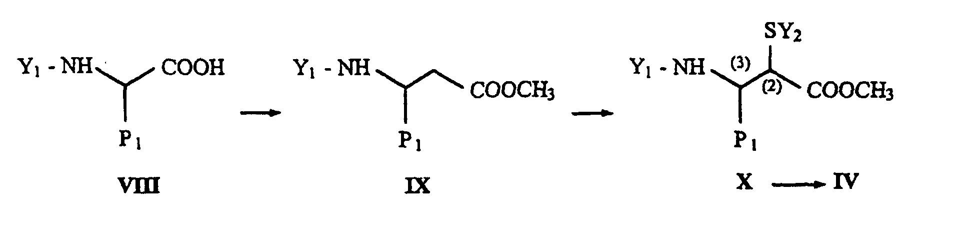

Un α-acide aminé protégé sur le N-terminal VIII est homologué par une méthode bien connue de l'homme de l'art en β-amino ester IX. Une réaction de sulfénylation par des réactifs décrits dans Shibata et al (Synlett. (1996)519-520 ; Tetrahedron 52(1996)12839-12852) ou dans Bischoff et al. (J. Org. Chem. 62(1997)4848-4850) en présence de LDA ou LiHMDS, conduit ensuite au dérivé X qui après hydrolyse alcaline de l'ester méthylique conduit au composé IV.

Les α-acides aminés N protégés VIII sont commerciaux ou peuvent être préparés dans des conditions énantiosélectives par la méthode d'Oppolzer (*Tetrahedron Lett*. 30(1989)6009-6010).

Une seconde voie de synthèse, illustrée par le schéma réactionnel suivant, a été utilisée.

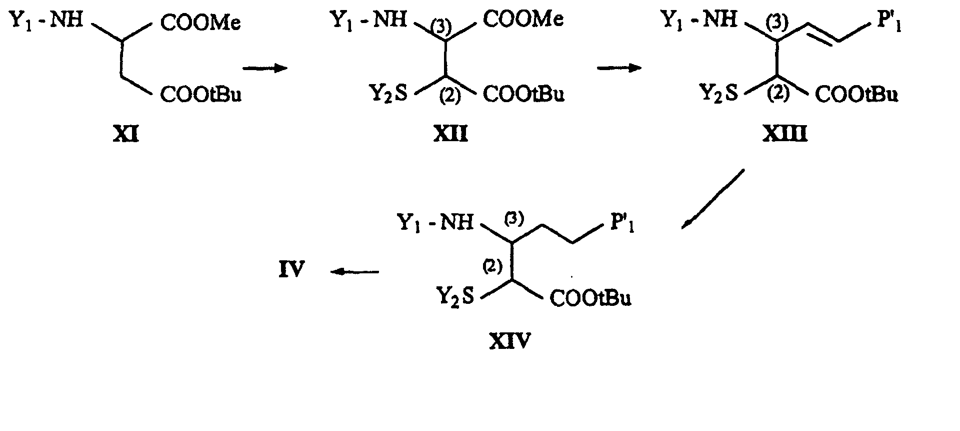

Cette seconde méthode de synthèse a pour avantage d'être diastéréosetective. Elle comprend les étapes consistant à :
― sulfényler le diester N protégé de formule XI de l'acide aspartique de configuration 2 donnée en un composé XII de configurations en positions 2 et 3 identiques mais inversées par rapport à la configuration du composé de formule XI,
― à réduire la fonction ester α en aldéhyde,
― à la condenser in situ avec un réactif organique de manière à obtenir un composé de formule générale XIII de même configuration et portant un groupement P'₁ qui est tel que le 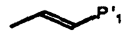 soit un précurseur du groupement P₁,
― à réduire ledit composé de formule XIII en composé XIV sous forme de 2 diastéréoisomères et
― à les hydrolyser en milieu acide pour obtenir le composé de formule IV attendu.

A titre illustratif de ce type de mode de synthèse on peut notamment proposer le suivant :

Le diester, N protégé XI de l'acide aspartique de configuration S, par exemple, est sulfénylé comme décrit dans la méthode précédente en composé XI de configuration 2R,3R. La fonction ester en α est réduite par le dibal (diisobutyl aluminium hydrure) en aluminoxyacétal puis est condensée in situ avec un dialkylphosphonate ou un ylure de phosphonium, conduisant ainsi au composé insaturé XII de même configuration. La réduction de XII par un hydrure de cuivre [(Ph₃P)CuH]₆ conduit à XIII, majoritairement de même configuration (d.e. 85%). La réduction de la double liaison par NaBH₄, par des dérivés du bore, tels que le diborane dans l'acide acétique, ou le cyclohexane en présence de Palladium conduit à XIII sous forme de 2 diastéréoisomères. Par hydrolyse acide, on obtient le composé IV.

Comme énoncé précédemment, le composé IV possède deux carbones asymétriques, C₂ et C₃. La stéréochimie de C₃ est définie par celle de l'acide aminé utilisé en début de synthèse (schéma 1 ou schéma 2) car elle n'est pas modifiée au cours des différentes étapes.

La réaction de sulfénylation est diastéréosélective et se fait en anti.

Au cours des étapes de réduction en milieu basique (XIII → XIV) une épimérisation sur le carbone C₂ au pied du soufre permet d'obtenir les stéréoisomères manquants qui sont séparés par chromatographie sur colonne après couplage du dipeptide ou par HPLC après couplage et déprotection partielle.

On obtient ainsi les quatre stéréoisomères du composé de formule générale IV. Selon un mode préféré de l'invention, on privilégie la formation de l'isomère 2R, 3R.

Les composés revendiqués s'avèrent capables d'inhiber significativement et sélectivement l'aminopeptidase A par rapport à l'aminopeptidase N.

Ce facteur de sélectivité est généralement supérieur à 10² et plus préférentiellement à 10³.

En conséquence, la présente invention a également pour objet l'utilisation des composés revendiqués à titre d'inhibiteurs sélectifs à l'égard de l'aminopeptidase A.

A ce titre, les composés revendiqués peuvent être utilisés en thérapie dans tous les processus pathologiques initiés par des substrats physiologiques de l'aminopeptidase A comme l'octapeptide C-terminal de la cholécystokinine CCK8 et l'angiotensine II et dont les rôles physiologiques ont été commentés précédemment Ils peuvent ainsi être utilisés pour diminuer la prise alimentaire, moduler des états d'anxiété ou de crises de panique ou traiter l'hypertension artérielle essentielle et secondaire, l'insuffisance cardiaque et rénale, les troubles de l'homéostasie hydrodynamique, l'infarctus du myocarde et la protéinurie chez les diabétiques.

A ces fins, les composés revendiqués et leurs dérivés peuvent être utilisés pour la préparation de compositions pharmaceutiques correspondantes.

Plus particulièrement, la présente invention concerne, selon un autre de ses aspects, une composition pharmaceutique comprenant en tant que principe actif au moins un composé de formule générale (I) ou un de ses dérivés.

Bien entendu, ce composé peut y être associé à au moins un véhicule pharmaceutiquement acceptable.

De même on peut envisager d'introduire conjointement deux ou plusieurs composés de formule générale I dans une même composition pharmaceutique.

Ces compositions pharmaceutiques peuvent être administrées par voie orale, parentérale, sublinguale, transdermique ou topique.

En ce qui concerne l'administration par voie orale ou sublinguale, on utilise en particulier des comprimés, simples ou dragéifiés, des gélules, des granules éventuellement à libération retardée, des gouttes ou encore des liposomes. En ce qui concerne l'administration par voie intraveineuse, sous-cutanée ou intramusculaire, on a recours à des solutions stériles ou stérilisables en particulier pour perfusion veineuse, tandis que l'on peut réaliser les patches conventionnels pour l'administration par voie transdermique.

Les compositions pharmaceutiques selon la présente invention peuvent être préparées selon des méthodes usuelles bien connues dans le domaine de la technique pharmaceutique.

Le principe actif peut être incorporé dans les excipients habituellement employés dans ces compositions pharmaceutiques, tels que le talc, la gomme arabique, le lactose, l'amidon, le stéarate de magnésium, les véhicules aqueux ou non, les corps gras d'origine animale ou végétale, les dérivés paraffiniques, les glycols, les différents agents mouillants, dispersants ou émulsifiants, les conservateurs, etc.

La quantité de principe actif à administrer par jour dépend bien entendu de la particularité de l'indication thérapeutique, de la gravité des affections à traiter, ainsi que du poids du malade et de la voie d'administration.

Pour une administration systémique, la dose globale chez l'homme varie généralement entre 1 et 100 mg par jour, par exemple de 2 à 50 mg, et plus convenablement de 3 à 40 mg par jour.

Des formes unitaires de dosage pour l'administration systémique comprendront généralement de 3 à 50 mg (à savoir 3, 5, 10, 20, 30, 40, et 50 mg de produit). Ces doses unitaires seront administrées normalement une ou plusieurs fois par jour, de préférence une à trois fois par jour.

Pour l'administration topique, les compositions pharmaceutiques contiennent généralement de 0,0001 à 1 % de principe actif et de préférence de 0,01 à 5 %.

Les composés décrits selon l'invention peuvent également être utilisés dans le cadre d'applications non thérapeutiques en particulier dans des systèmes de diagnostic et de mesure de l'expression de l'aminopeptidase A.

La présente invention a également pour objet un système de diagnostic pour détecter et doser l'aminopeptidase A caractérisé en ce qu'il comprend au moins un composé de formule générale I.

Les exemples présentés ci-après à titre non limitatif mettront en évidence d'autres avantages de la présente invention.

### EXEMPLE - 1 -

### N-[[(2S,3R) et (2R,3R), 3-amino, 2-sulfhydryl, 5-sulfonate] pentanoyl]-L.Tyr-L.Sal-OH

### ETAPE 1.

### Acide (2R,3R), 3-benzyloxycarbonylamino, 2-(4-méthoxybenzyl sulfanyl)succinate de 4-méthyle et de tertbutyle.

Sous argon, à une solution de 1,1,1,3,3,3-hexamethyldisilazane (36 ml ; 171 mmol) dans le tetrahydrofuranne sec (375 ml) est ajoutée, goutte à goutte à 0°C une solution de n-butyl lithium 2,5 M dans n-hexane (56 ml ; 140 mmol) et le milieu est agité ∼15 min à 0°C. La solution est refroidie à -78°C et le composé Z-L.Asp(OtBu)-OMe (20,7 g ; 61 mmol) dans le THF sec (255 ml) est ajouté goutte à goutte. Après agitation du mélange réactionnel pendant 2 h entre -40°C et -30°C, le milieu est refroidi à -78°C et le disulfure de 4-méthoxybenzyle et de 2,4-dinitrophényle (30 g ; 85,4 mmol) solide est ajouté. Après 1 h à -78°C, le milieu est hydrolysé par HCl 1N (200 ml) et le produit extrait par 500 ml d'Et₂O. La phase organique est lavée par 100 ml d'acide citrique, 100 ml H₂O, 100 ml NaCl saturé, séchée sur Na₂SO₄ et évaporée à sec. Le résidu est repris par de l'Et₂O froid et le précipité d'agent de sulfénylation en excès est filtré. Le filtrat après évaporation à sec, est purifié par flash chromatographie sur gel de silice (éluant cHex, AE, CH₂Cl₂ 8 : 1 : 1, R_{f} = 0,19) donnant des cristaux jaunes, 20 g (68 %) (ee : 95/5). HPLC C₁₈ Kromasil (5 µ, 100 Å) isocratique CH₃CN/H₂O (TFA) 75 : 25, t_{R} = 9,9 min, P_{f} = 71,6-73,3°C.

### ETAPE 2.

### (2R,3R), 3-benzyloxycarbonylamino, 2-(4-méthoxybenzyl sulfanyl), 5-(2,2-diméthylpropanoxysulfonyl), pent-4-énoate de tert.butyle.

A une solution de diéthylphosphonométhane sulfonate de néopentyle (2,5 g ; 8,2 mmol) dans THF sec (37 ml) à -78°C sous argon, est ajoutée goutte à goutte, une solution de n-butyl lithium 2,5 M dans hexane (3,5 ml ; 8,6 mmol). Le mélange est agité pendant 30 min à -78°C puis une solution du composé de l'étape 1 (2 g ; 4,1 mmol) dans le THF sec (4 ml) est ajoutée, suivi, goutte à goutte d'une solution de Dibal-H 1,5 M dans le toluène (5,3 ml ; 8 mmol). Après agitation 4 h à -78°C et 1 heure à température ambiante, le mélange réactionnel est hydrolysé par H₂O (8 ml) et HCl 2N (8 ml). La phase aqueuse est alors extraite par 160 ml d'AE. La phase organique est lavée par NaCl saturé, séchée sur Na₂SO₄ et le solvant évaporé à sec. Le produit pur est obtenu après flash chromatographie (éluant cHex, AE, CH₂Cl₂ 7 : 1.5 : 1.5, R_{f} = 0,39) sous forme d'une huile jaune, 1,6 g (68 %). HPLC C₁₈ Kromasil (5 µ, 100 Å) isocratique CH₃CN/H₂O (TFA) 75 : 25 t_{R} = 17,1 min. SM (Electrospray): 629,8 = MNa⁺.

### ETAPE 3.

### • Procédure 1.

### (2RS,3R), 3-benzyloxycarbonylamino, 2-(4-méthoxybenzyl sulfanyl), 5-(2,2-diméthylpropanoxysulfonyl)pentanoate de tert.butyle.

L'éthylénique obtenu dans l'étape 2 (10 g ; 16,4 mmol) dans EtOH (164 ml) est réduit par NaBH₄ (996 mg ; 26,3 mmol) pendant 3 h à température ambiante. Après un traitement classique, le produit est obtenu sous forme d'une huile orange, 10 g (99 %). R_{f}(cHex, CH₂Cl₂, AE 6 : 2 : 2) = 0,50. Il présente une épimérisation totale sur le C₂. HPLC C₁₈ Kromasil (5 µ, 100 Å) isocratique CH₃CN/H₂O (TFA) 75: 25 t_{R} = 15,9 min et 16,6 min.

### • Procédure 2.

### (2R,3R), 3-benzyloxycarbonylamino, 2-(4-méthoxybenzyl sulfanyl), 5-(2,2-diméthylpropanoxysulfonyl)pentanoate de tert.butyle.

Une solution de l'éthylénique précédent (100 mg ; 0,16 mmol) dans le benzène (2,5 ml) est dégazée sous argon pendant 15 minutes. A cette solution sont ajoutés successivement à température ambiante 60 µl (3,3 mmol) d'eau puis 120 mg (0,06 mmol) de [(Ph₃P)CuH]₆. Le milieu réactionnel est agité pendant 3 heures à température ambiante puis hydrolysé par une solution de NH₄Cl, et extrait par l'AE. La phase organique est lavée par NaCl saturée, séchée sur Na₂SO₄ et évaporée à sec donnant le produit (80 mg ; 80 %) sous forme d'huile jaune présentant un excès énantiomérique de 85 : 15. HPLC C₁₈ Kromasil (5 µ, 100 Å) isocratique CH₃CN/H₂O (TFA) 75 : 25 t_{R} = 16,6 min.

### ETAPE 4.

### Acide (2RS, 3R), 3-benzyloxycarbonylamino, 2-(4-méthoxybenzyl sulfanyl), 5-(2,2-diméthylpropanoxysulfonyl) pentanoïque.

Une solution du composé de l'étape 3 (10 g ; 16,4 mmol) dans le CH₂Cl₂ (13 ml) est traitée par anisole (630 µl ; 5 % vol.) et le TFA (12,6 ml ; 164 mmol) et conduit après 3 heures d'agitation à température ambiante, à l'acide attendu qui est chromatographié (gel de silice, flash, éluant cHex, Et₂O, HCOOH 4 : 6 : 0,1, R_{f} = 0,40) donnant une mousse rouge, 6,1 g (69 %). HPLC C₁₈ Kromasil (5 µ, 100 Å) isocratique CH₃CN/H₂O (TFA) 75 : 25 t_{R} = 5,6 min.

### ETAPE 5.

### N-[(2S,3R) et (2R,3R), 3-benzyloxycarbonylamino, 5-[2,2-diméthylpropanoxysulfonyl], 2-[4-méthoxybenzylsulfanyl]pentanoyl]-L.Tyr(t.Bu)-L.Sal(OCH₂tBu)OtBu

L'acide est couplé en phase solide avec le dipeptide H-Tyr(tBu)-Sal(OCH₂tBu) greffé sur une résine de type Wang en utilisant l'agent de couplage BOP et de la diisopropyléthylamine.

### ETAPE 6.

### N-[(2R,3R) et (2S,3R), 3-benzyloxycarbonylamino, 5-[2,2-diméthylpropanoxy sulfonyl], 2-[4-méthoxybenzylsulfanyl]pentanoyl]-L.Tyr-L.Sal-OH.

La peptidyl résine dans CH₂Cl₂ (2 ml) est traitée à 0°C avec 60 µl d'anisole (5 % vol.) et l'acide trifluoroacétique (1,2 ml ; 15 mmol) et le mélange réactionnel est agité pendant 3,5 heures à température ambiante. Après évaporation à sec, le résidu est repris par de l'Et₂O froid et le précipité obtenu est tritué dans le même solvant. Le produit est récupéré après décantation de la phase éthérée sous forme d'une poudre blanche.

### ETAPE 7.

### N-[[(2RS,3R), 3-amino, 2-sulfhydryl, 5-sulfonate]pentanoyl]-L.Tyr-L.Sal-OH.

Le pseudo-tripeptide de l'étape 6 (0,43 mmol) est traité par 500 µl de méta-crésol distillé extemporanément, puis à -78°C, 10 ml de fluorure d'hydrogène liquide. Après agitation pendant 1 heure à 0°C, le HF est évaporé sous vide. Le résidu est repris par 2 x 1 ml de TFA et le produit est obtenu par précipitation dans Et₂O/n-hexane froid et centrifugation. Le composé est enfin purifié par HPLC semi-préparative C₁₈ Kromasil (10 µ, 100 Å) en gradient CH₃CN/H₂O (TFA) de 10 à 60%, t_{R} = 4,1 min, donnant une poudre blanche (20 mg). SM (Electrospray) : 544,6 = MH⁺, 566,6 = MNa⁺.

### EXEMPLE - 2 -

### N-[[(2S,3R) et (2R,3R), 3-amino, 2-sulfhydryl, 5-sulfonate] pentanoyl]-L.Tyr-L.hSal-OH

Selon le procédé utilisé dans l'exemple 1 avec le dipeptide H-Tyr(tBu)-hSal(OCH₂tBu) greffé sur une résine Wang, les deux stéréoisomères séparés sont obtenus sous forme de 2 poudres blanches (14 et 17 mg). HPLC C₈ Kromasil (5 µ, 100 Å) gradient CH₃CN/H₂O (TFA) de 10 à 60 %, t_{R} = 2,8 min et 3,4 min. SM (Electrospray): 557,9 = MH⁺, 579,9 = MNa⁺.

### EXEMPLE - 3 -

### N-[[(2S,3R) et (2R,3R), 3-amino, 5-carboxy, 2-sulfhydryl] pentanoyl]-L.Ile-L.(3R)(3-COOH)Pro-OH

Ce composé est obtenu en suivant le protocole décrit pour l'exemple 1, en utilisant dans l'étape 1 le Z-L-Asp (OtBu)OMe dans l'étape 5 le dipeptide Ile-(3R)(3-CO₂CH₂Ph)ProOCH₂Ph et en omettant l'étape 6.

3A: [[(2S,3R), 3-amino, 5, carboxy, 2-sulfhydryl]pentanoyl]-L.Ile-L.(3R)(3-COOH)Pro-OH
Poudre blanche (5 mg). HPLC C₁₈ Kromasil (5µ, 100 Å) CH₃CN/H₂O (TFA) 15/85, t_{R}= 8,5 min. SM (Electrospray) : 448,6 = MH^{+.}

3B : [[(2R,3R), 3-amino, 5-carboxy, 2-sulfhydryl]-pentanoyl]-L.Ile-L.(3R)(3-COOH)Pro-OH
Poudre blanche (10 mg). HPLC C₁₈ Kromasil (5µ, 100 Å) CH₃CN/H₂O (TFA) 15/85, t_{R}= 6,0 min. SM (Electrospray) : 448,0 = MH⁺.

### EXEMPLE - 4 -

### N-[[(2S,3R) et (2R,3R), 3-amino, 5-phosphonate, 2-sulfhydryl] pentanoyl]-L.Ile-L.Glu-OH

Ce composé est obtenu en suivant le protocole décrit pour l'exemple 1, en utilisant le tétrabenzyl diphosphoryl méthylène dans l'étape 2 et le dipeptide Ile-Glu(OtBu)OtBu dans l'étape 5.

4A : [[(2S,3R),3-amino, 5-phosphonate, 2-sulfhydryl]-pentanoyl]-L.Ile-L.Glu-OH
Poudre blanche (23 mg). HPLC C₁₈ Kromasil (5µ, 100 Å) CH₃CN/H₂O (TFA) 15/85, t_{R}= 6,97 min. SM (Electrospray) : 472,4 = MH⁺.

4B : [[(2R,3R),3-amino, 5-phosphonate, 2-sulfhydryl]-pentanoyl]-L.Ile-L.Glu-OH
Poudre blanche (33 mg). HPLC C₁₈ Kromasil (5µ, 100 Å) CH₃CN/H₂O (TFA) 15/85, t_{R}= 4,6 min. SM (Electrospray) : 472,5 = MH⁺.

### EXEMPLE - 5 -

### N-[(2S,3R) et (2R,3R), 3-amino, 2-sulfhydryl, 5-sulfonate] pentanoyl]-L.Ile-L.Sal-OH

Ce composé est obtenu en suivant le protocole décrit pour l'exemple 1 en utilisant dans l'étape 5 le dipeptide Ile-Sal(CH₂tBu)OH.

5A : [[(2S,3R), 3-amino, 2-sulfhydryl, 5-sulfonate]-pentanoyl]-L.Ile-L.Sal-OH
Poudre blanche (4 mg). HPLC C₁₈ Kromasil (5µ, 100 Å) CH₃CN/H₂O (TFA) 15/85, t_{R}= 4,1 min. SM : 493,9 = MH⁺.

5B : [[(2R,3R), 3-amino, 2-sulfhydryl, 5-sulfonate]-pentanoyl]- L.Ile-L.Sal-OH
Poudre blanche (13 mg). HPLC C₁₈ Kromasil (5µ, 100 Å) CH₃CN/H₂O (TFA) 15/85, t_{R}= 2,4 min.

### EXEMPLE - 6 -

### N-[[(2S,3R) et (2R,3R), 3-amino, 2-sulfhydryl, 5-sulfonate] pentanoyl]-L.Ile-L.(3R)(3-COOH)Pro-OH

Ce composé est obtenu en suivant le protocole décrit pour l'exemple 1 en utilisant dans l'étape 5 le dipeptide Ile-(3R)(3-CO₂CH₂Ph)Pro OCH₂Ph et en omettant l'étape 6.

6A : [[(2S,3R), 3-amino, 2-sulfhydryl-5, sulfonate]-pentanoyl]- L.Ile-L.(3R)(3-COOH)Pro-OH
Poudre blanche (19 mg). HPLC C₁₈ Kromasil (5µ, 100 Å) CH₃CN/H₂O (TFA) 15/85, t_{R}= 9,9 min. SM : 484,1 = MH⁺, 506,1 =MNa⁺.

6B : [[(2R,3R), 3-amino, 2-sulfhydryl, 5-sulfonate]-pentanoyl]- L.Ile-L.(3R)(3-COOH)Pro-OH
Poudre blanche (33 mg). HPLC C₁₈ Kromasil (5µ, 100 Å) CH₃CN/H₂O (TFA) 15/85, t_{R}= 3,6 min. SM : 484,1 = MH⁺.

### EXEMPLE - 7 -

### N-[[(2S,3R) et (2R,3R), 3-amino, 2-sulfhydryl, 5-sulfonate] pentanoyl]-L.Ile-L.(3S)(3-COOH)Pro-OH

Ce composé est obtenu en suivant le protocole décrit pour l'exemple 1 en utilisant dans l'étape 5 le dipeptide Ile-(3S)(3-CO₂CH₂Ph)ProOCH₂Ph et en omettant l'étape 6.

7A : [[(2S,3R), 3-amino, 2-sulfhydryl, 5-sulfonate]-pentanoyl]-L.Ile-L.(3S)(3-COOH)Pro-OH
Poudre blanche (4 mg). HPLC C₁₈ Kromasil (5µ, 100 Å) CH₃CN/H₂O (TFA) 15/85, t_{R}= 14,9 min. SM : 484,5 = MH⁺, 522,5 = MK⁺.

7B : [[(2R,3R), 3-amino, 2-sulfhydryl, 5-sulfonate]-pentanoyl]-L.Ile-L.(3S)(3-COOH)Pro-OH
Poudre blanche (6 mg). HPLC C₁₈ Kromasil (5µ, 100 Å) CH₃CN/H₂O (TFA) 15/85, t_{R}= 4,7 min. SM : 484,5 = MH⁺, 522,3 = MK⁺.

### EXEMPLE - 8 -

### N-[[(2S,3R) et (2R,3R), 3-amino, 2-sulfhydryl, 5-sulfonate] pentanoyl]-L.Ile-L.Glu-NH₂

En suivant les étapes 1 à 7 décrites dans l'exemple 1 mais en couplant avec le dipeptide H-Ile-Glu(OtBu)-NH₂, sont obtenus les deux stéréoisomères du composé désiré, sous forme de 2 poudres blanches (20 mg et 10 mg). HPLC C₁₈ Kromasil (5 µ, 100 Å) isocratique CH₃CN/H₂O (TFA) 15/85, t_{R} = 3,5 min et 4,9 min. SM (Electrospray): 470,9 = MH⁺.

### Diéthyl phosphoryl acétate de benzyle ou (diéthoxy-phosphoryl)-acétate de benzyle.

Ce réactif est synthétisé selon la procédure décrite par Jennings et coll. (1992).
Tétrabenzyl diphosphoryl méthylene ou (dibenzyloxy-phosphoryl-méthyl)-phosphonate de dibenzyle.

Ce réactif est synthétisé selon la procédure décrite par Saady et coll. (1995).

### EXEMPLE - 9 -

### ETUDE IN VITRO DU POUVOIR INHIBITEUR DES COMPOSES SYNTHETISES SUR L'AMINOPEPTIDASE A ET L'AMINOPEPTIDASE N.

### a) Etude in vitro du pouvoir inhibiteur des composés synthétisés sur l'aminopeptidase A.

### 1°) Purification de l'enzyme.

L'aminopeptidase A est obtenue à partir de rein de lapin comme décrit dans "*Neuropeptides 16 (1990)163-168*". Le substrat synthétique utilisé est le L-glutamyl-β-naphthylamide (Glu NA, Km = 130 µM). L'enzyme purifiée présente une activité spécifique vis à vis de Glu NA de 100 µmol.mL⁻¹.li⁻¹.

### 2°) Méthode de dosage.

Le procédé décrit par Goldbarg "*Cancer 11 (1958)283-291*" a été transposé à l'échelle de la microplaque.

L'APA est incubée pendant 1 h à 37°C en présence de 200 µM Glu NA et en présence de concentrations variables d'inhibiteurs dans le tampon Tris HCl (50 mM), pH = 7,4, 4 mM CaCl₂ (volume final 100 µl). La réaction est stoppée par addition de 10 µl d'HCl 3N. On ajoute alors dans chaque puit 25 µl de NaNO₂ (87 mM), puis, 3 min après, 50 µl de sulfamate d'ammonium (0,13 M). Enfin, au bout de 5 min, on ajoute 25 µl d'une solution 23 mM de 1-naphthyléthylene diamine à 37°C. L'absorbance à 560 nm est mesurée et comparée à celle d'une gamme étalon standard de 2-naphthylamine.

### b) Etude in vitro du pouvoir inhibiteur des composés synthétisés sur l'aminopeptidase N.

### 1°) Source de l'enzyme.

L'aminopeptidase N purifié à partir du rein de porc est commercialisé chez Bôehringer Mannheim (Meylan, France).

### 2°) Méthode de dosage.

L'APN est incubée pendant 10 min à 37°C en absence ou en présence de concentrations croissantes d'inhibiteurs dans un volume total de 100 µl dans le tampon Tris HCl (50 mM, pH = 7,4. On ajoute 200 µM de Ala-Na et on incube 30 min à 37°C. La réaction est stoppée par addition de 10 µl CH₃COO-Na 1M (pH = 4,2). L'absorbance est mesurée à 400 nM et comparée à celle d'une gamme étalon de 2-naphthylamine.

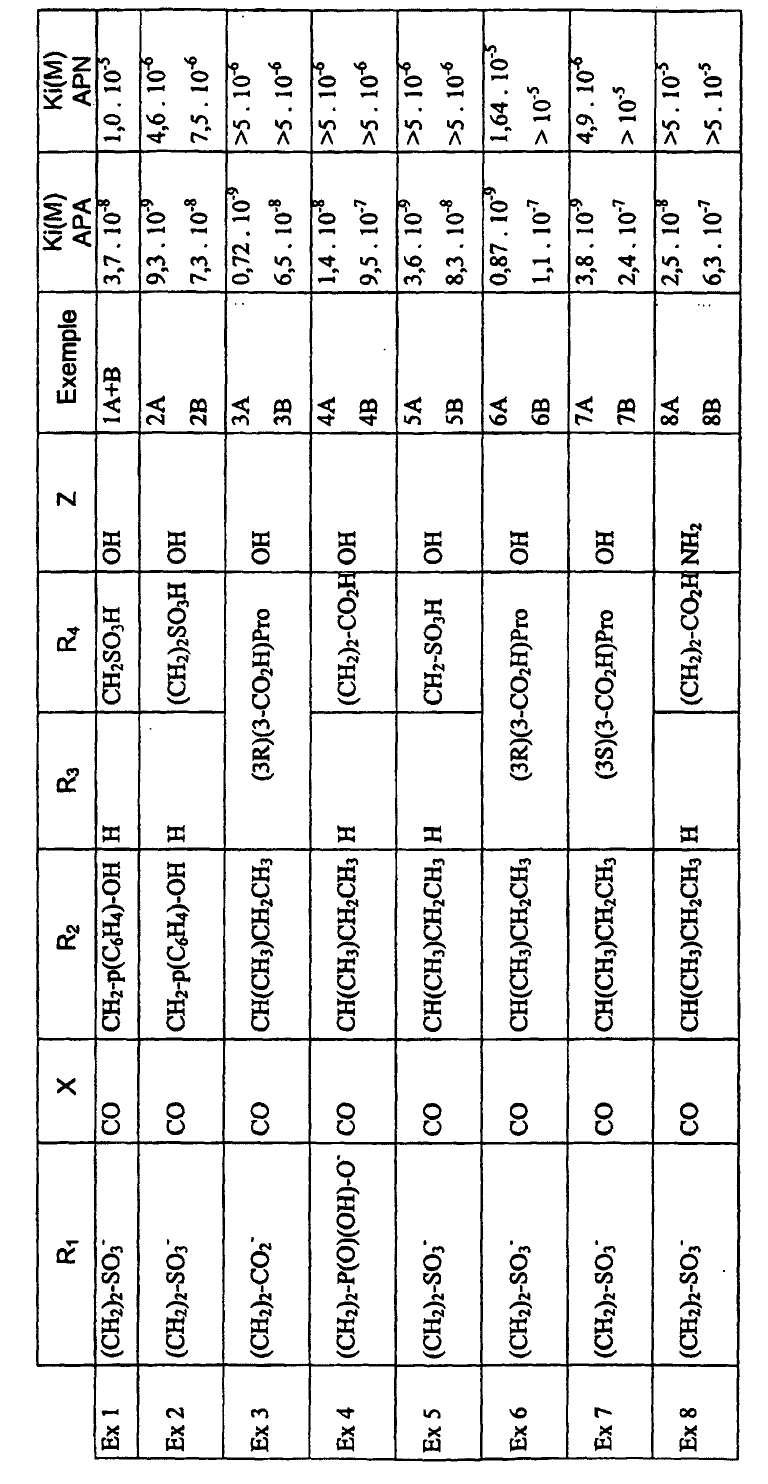

## Revendications

1. Composé de formule générale I : 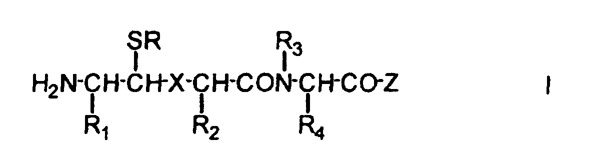 dans laquelle :
• R₁ représente une chaîne alkyle, alkényle ou alkynyle, ou un groupement cycloalkyle, ou (cycloalkyle)alkyle substituée par au moins un groupement
― -COOH, éventuellement estérifié par un groupe alkyle comportant de 2 à 12 atomes de carbone,
― SO₃H, éventuellement protégé par un groupement pentylique,
― PO₃H₂, éventuellement substitué par un groupement (-CH₂CH₂SCOR₅) avec R₅ représentant un groupement alkyle en C₁-C₄, un groupement phényle ou benzyle, ou
― tétrazolyle.
• R₂ représente une chaîne alkyle, ou un groupement aryle, arylalkyle, cycloalkyle, (cycloalkyle)alkyle, (héteroaryl)alkyle substituée ou non par au moins un groupement OH, OR, SR', NH₂, NHR', guanidinyle, COOH, CONH₂, ou un atome d'halogène choisi parmi le F, Cl, Br ou I avec R' représentant un alkyle en C₁ à C₄ linéaire ou ramifié.
• R₃ représente un atome d'hydrogène ou un groupement méthyle,
• R₄ représente
― une chaîne alkyle, un groupement aryle, arylalkyle, cycloalkyle, (cycloalkyl)alkyle, (hétéroaryl)alkyle, hétérocycloalkyle ou (hétérocycloalkyl)alkyle substituée par au moins un groupement CONH₂, SO₃H, SO₂NH₂, PO₃H₂ ou tétrazolyle, avec le groupement SO₃H pouvant être protégé par un groupement pentylique, et le groupement PO₃H₂ pouvant être protégé par un groupement (-CH₂CH₂SCOR₅) avec R₅ représentant un groupement alkyle en C₁-C₄, un groupement phényle ou benzyle,
― un groupement aryle, arylalkyle, cycloalkyle, (cycloalkyl)alkyle, (hétéroaryl)alkyle, hétérocycloalkyle ou (hétérocycloalkyl)alkyle substituée par au moins un groupement CO₂H pouvant être protégé comme décrit ci-dessus, à savoir éventuellement estérifié par un groupe alkyle comportant de 2 à 12 atomes de carbone, ou
• R₃ et R₄ peuvent constituer ensemble un composé hétérocyclique, à 5 ou 6 chaînons, substitué par au moins un groupement CO₂H, CONH₂, SO₃H, SO₂NH₂ ou PO₃H₂ avec les groupements CO₂H, SO₃H et PO₃H₂ le cas échéant protégés comme décrit ci-dessus,
• X représente un groupement CONH ou CH₂NH,
• Z représente un groupement OH, OCH₂-C₆H₅ ou NR"R''' dans lequel R" et R'''' peuvent représenter indépendamment l'un de l'autre un atome d'hydrogène ou un groupement alkyle, aryle ou arylalkyle, avec R" et R"' pouvant constituer ensemble, avec l'atome d'azote, un hétérocycle à 5 ou 6 chaînons possédant le cas échéant un 2^{ème} hétéroatome choisi parmi O, S et N et
• R représente un atome d'hydrogène ou un groupement de formule II 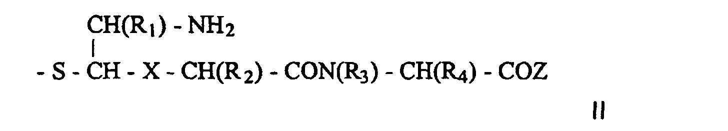 correspondant au disulfure symétrique de l'inhibiteur avec R₁, R₂, R₃, R₄, X et Z étant tels que définis ci-dessus,
leurs sels d'addition obtenus avec des acides organiques ou minéraux pharmacologiquement acceptables,
et les dimères de composés de formule (I), constitués par deux molécules de composés de formule (I), identiques ou différents couplées entre elles au niveau de leurs atomes de soufre respectif, R représentant alors un groupement de formule (II) tel que défini ci-dessus.

2. Composé selon la revendication 1, **caractérisé en ce que**
• R₄ représente une chaîne alkyle, un groupement aryle, arylalkyle, cycloalkyle, (cycloalkyl)alkyle, (hétéroaryl)alkyle, hétérocycloalkyle ou (hétérocycloalkyl)alkyle substituée par au moins un groupement CONH₂, SO₃H, SO₂NH₂, PO₃H₂ ou tétrazolyle, avec les groupements SO₃H et PO₃H₂ pouvant être protégés, ou
• R₄ constitue avec R₃ un composé hétérocyclique, à 5 ou 6 chaînons, substitué par au moins un groupement CO₂H, CONH₂, SO₃H, SO₂NH₂ ou PO₃H₂ avec les groupements CO₂H, SO₃H et PO₃H₂ pouvant être protégés comme dècrit dans la revendication 1.

3. Composé selon la revendication 1 ou 2, **caractérisé en ce que** R₄ et R₃ constituent ensemble un composé hétérocyclique, à 5 ou 6 chaînons, substitué par au moins un groupement CO₂H, CONH₂, SO₃H, SO₂NH₂ ou PO₃H₂ avec les groupements CO₂H, SO₃H et PO₃H₂ pouvant être protégés comme dècrit dans la revendication 1.

4. Composé selon l'une des revendications précédentes, **caractérisé en ce que** X représente une fonction CONH.

5. Composé selon l'une des revendications précédentes, **caractérisé en ce que** R₂ représente une chaîne alkyle ou arylalkyle substituée le cas échéant.

6. Composé selon au moins l'une des revendications 1 à 5, **caractérisé en ce qu'**il est choisi parmi :
les N-[[(2S,3R) et (2R,3R), 3-amino, 2-sulfhydryl, 5-sulfonate] pentanoyl]-L.Tyr-L.Sal-OH ; et
les N-[[(2S,3R) et (2R,3R), 3-amino, 2-sulfhydryl, 5-sulfonate] pentanoyl]-L.Tyr-L.hSal-OH.

7. Procédé de préparation d'un composé de formule générale I 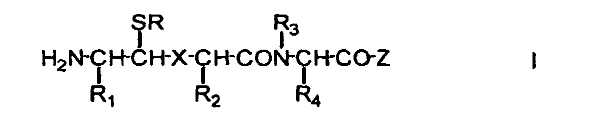 avec R₁, R₂, R₃, R₄, R et Z tel que défini en revendication 1 ou 2 et X représentant un groupement CONH, **caractérisé en ce qu'**il comprend au moins le couplage d'un dipeptide ester de formule générale III 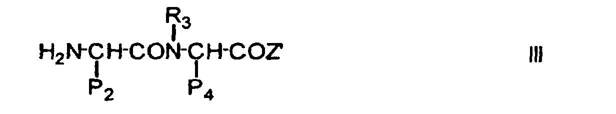 dans laquelle
― P₂ et P₄ correspondent à des formes protégées de R₂ et R₄,
― Z' représente un groupement OC(CH₃)₃, OCH₂-C₆H₅ ou NR"R"' dans lequel R" et R''' peuvent représenter indépendamment l'un de l'autre un atome d'hydrogène ou un groupement alkyle, aryle ou arylalkyle, avec R" et R''' pouvant constituer ensemble, avec l'atome d'azote, un hétérocycle à 5 ou 6 chaînons possédant le cas échéant un 2^{ème} hétéroatome choisi parmi O, S et N,
avec un composé de formule générale IV, 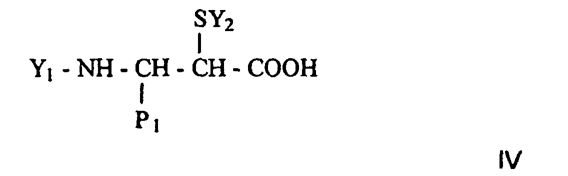 dans laquelle :
― Y₁ représente un groupement protecteur
― Y₂ représente un groupement protecteur
― P₁ représente une forme protégée de R₁,
dans des conditions suffisantes pour conduire via le composé V 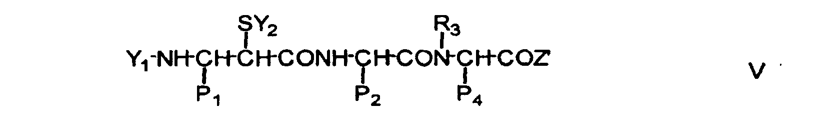 et sa déprotection audit composé de formule générale I, la réaction de couplage étant réalisée dans un solvant organique, en présence d'un agent de couplage et d'une amine tertiaire et à une température de l'ordre de 20°C.

8. Procédé de préparation d'un composé de formule générale 1 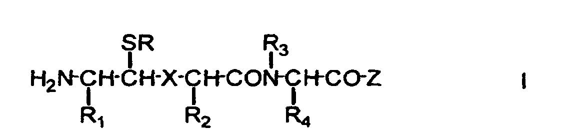 dans laquelle R₁, R₂, R₃, R₄, R et Z sont tels que définis en revendication 1 et X représente un groupement CH₂-NH, **caractérisé en ce qu'**il comprend au moins :
― la condensation d'un composé de formule générale III 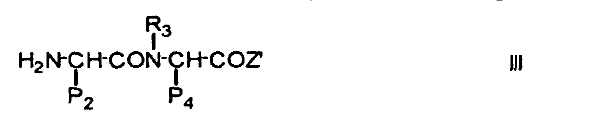
― Z' représente un groupement OC(CH₃)₃, OCH₂-C₆H₅ ou NR"R''' dans lequel R" et R''' peuvent représenter indépendamment l'un de l'autre un atome d'hydrogène ou un groupement alkyle, aryle ou arylalkyle, avec R" et R''' pouvant constituer ensemble, avec l'atome d'azote, un hétérocycle à 5 ou 6 chaînons possédant le cas échéant un 2^{ème} hétéroatome choisi parmi O, S et N,
avec un composé de formule générale VI, 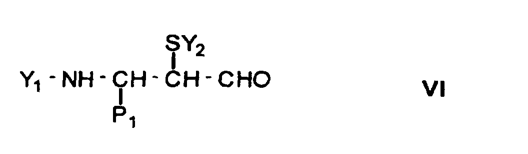 dans laquelle Y₁, Y₂ et P₁ sont tels que définis en revendication 6,
― la réduction de l'intermédiaire ainsi formé pour conduire via le composé de formule générale VII 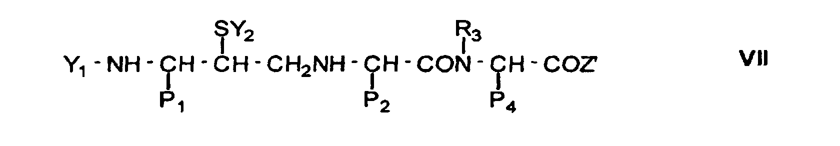 et sa déprotection audit composé de formule générale I.

9. Procédé selon l'une des revendications 7 ou 8, **caractérisé en ce que** les deux carbones asymétriques du dipeptide ester de formule générale III possèdent une configuration S.

10. Utilisation d'un composé tel que défini selon l'une des revendications 1 à 6 à titre d'inhibiteur sélectif à l'égard de l'aminopeptidase A.

11. Utilisation d'un composé tel que défini selon l'une des revendications 1 à 6 pour préparer un médicament destiné à diminuer la prise alimentaire, moduler des états d'anxiété ou de crises de panique ou traiter l'hypertension artérielle essentielle et secondaire, l'insuffisance cardiaque et rénale, des troubles de l'homéostasie hydrodynamique, l'infarctus du myocarde ou la protéinurie chez les diabétiques.

12. Composition pharmaceutique comprenant en tant que principe actif au moins un composé selon l'une des revendications 1 à 6.

13. Système de diagnostic pour détecter et doser l'aminopeptidase A **caractérisé en ce qu'**il comprend au moins un composé tel que défini à l'une des revendications 1 à 6.

14. Composé choisi parmi les N-[[(2S,3R) et (2R,3R), 3-amino, 5-carboxy, 2-sulfhydryl] pentanoyl]-L.Ile-L.(3R)(3-COOH)Pro-OH.

15. Utilisation d'un composé tel que défini selon la revendication 14, à titre d'inhibiteur sélectif à l'égard de l'aminopeptidase A.

16. Utilisation d'un composé tel que défini selon la revendication 14, pour préparer un médicament destiné à diminuer la prise alimentaire, moduler des états d'anxiété ou de crises de panique ou traiter l'hypertension artérielle essentielle et secondaire, l'insuffisance cardiaque et rénale, des troubles de l'homéostasie hydrodynamique, l'infarctus du myocarde ou la protéinurie chez les diabétiques.

17. Composé choisi parmi les N-[[(2S,3R) et (2R,3R), 3-amino, 5-phosphonate, 2-sulfhydryl] pentanoyl]-L.Ile-L.Glu-OH et les N-[[(2S,3R) et (2R,3R), 3-amino, 2-sulfhydryl, 5-sulfonate] pentanoyl]-L.Ile-L.Glu-NH₂.

18. Utilisation d'un composé tel que défini selon la revendication 17, à titre d'inhibiteur sélectif à l'égard de l'aminopeptidase.

19. Utilisation d'un composé tel que défini selon la revendication 17, pour préparer un médicament destiné à diminuer la prise alimentaire, moduler des états d'anxiété ou de crises de panique ou traiter l'hypertension artérielle essentielle et secondaire, l'insuffisance cardiaque et rénale, des troubles de l'homéostasie hydrodynamique, l'infarctus du myocarde ou la protéinurie chez les diabétiques.

20. Composé selon l'une quelconque des revendications 1 à 5, dans lequel le groupement pentylique protégeant SO₃H est un néopentyle.

## Patentansprüche

1. Verbindung der allgemeinen Formel 1: 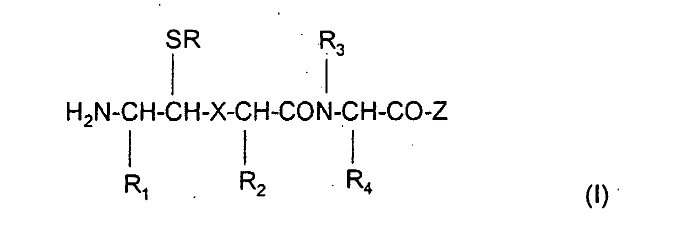 wobei:
R₁ eine substituierte Alkyl-, Alkenyl- oder Alkynylkette, oder eine substituierte Cycloalkyl- oder eine (Cycloalkyl)-Alkylgruppe, darstellt, die mit wenigstens einer funktionellen Gruppe aus
- -COOH, ggf. mit einer Alkylgruppe verestert, die 2 bis 12 Kohlenstoffatome umfasst,
- SO₃H, ggf. geschützt mit einer Pentyl-Gruppe,
- PO₃H₂, ggf. substituiert mit einer Gruppe (-CH₂CH₂SCOR₅), wobei R₅ eine C₁- bis C₄-Alkylgruppe, eine Phenyl- oder Benzylgruppe darstellt oder
- Tetrazolyl,
substituiert sind.
R₂ eine Alkylkette darstellt, oder eine Aryl-, Aralkyl-, Cycloalkyl-, (Cycloalkyl)-Alkyl-, (Heteroaryl)-Alkylgruppe, die ggf. mit mindestens einer OH, OR, SR', NH₂, NHR', Guanidinyl-, COOH-, CO₂NH₂-Gruppe substituiert ist oder mit einem Halogenatom, ausgewählt aus F, Cl, Br oder I, wobei R' ein lineares oder verzweigtes C₁- bis C₄-Alkyl darstellt,
R₃ ein Wasserstoffatom oder eine Methylgruppe darstellt,
R₄ ist
- eine Alkylkette, eine Aryl-, Arylalkyl-, Cycloalkyl-, (Cycloalkyl)-Alkyl-, (Heteroaryl)-Alkyl-, Heterocycloalkyl- oder (Heterocycloalkyl)-Alkylgruppe, die mit wenigstens einer CONH₂-, SO₃H-, SO₂NH₂-, PO₃H₂-Gruppe oder Tetrazolyl substituiert ist, wobei die Gruppe SO₃H durch eine Pentyl-Gruppe geschützt sein kann und die PO₃H₂-Gruppe mit einer (-CH₂CH₃SCOR₅) geschützt sein kann, wobei R₅ eine C₁- bis C₄-Alkylgruppe, eine Phenyl- oder Benzylgruppe darstellt,
- eine Aryl-, Arylalkyl-, Cycloalkyl-, (Cycloalkyl)-Alkyl-, (Heteroaryl)-Alkyl-, Heterocycloalkyl- oder (Heterocycloalkyl)-Alkylgruppe, die mit wenigstens einer CO₂H-Gruppe substituiert ist, die wie vorstehend beschrieben geschützt sein kann, insbesondere eventuell mit einer Alkylgrupe verestert sein kann, die 2 bis 12 Kohlenstoffatome umfasst, oder
R₃ und R₄ können zusammen eine heterocyclische Verbindung mit 5 oder 6 Ringgliedern bilden, die mit wenigstens einer CO₂H-, CONH₂-, SO₃H-, SO₂NH₂- oder PO₃H₂-Gruppe substituiert ist, wobei die Gruppen CO₂H, SO₃H und PO₃H₂ ggf. wie vorstehend beschrieben geschützt sind,
X eine CONH- oder CH₂NH-Gruppe darstellt,
Z eine OH-, OCH₂-C₆H₅- oder NR"R"'-Gruppe darstellt, wobei R" und R''' unabhängig voneinander ein Wasserstoffatom oder eine Alkyl-, Aryl- oder Arylalkylgruppe darstellen können, wobei R" und R''' zusammen mit dem Stickstoffatom einen Heterocyclus mit 5 oder 6 Ringgliedem bilden können, der ggf. ein zweites Heteroatom aufweist, das aus O, S und N ausgewählt ist und
R ein Wasserstoffatom oder eine Gruppe mit der Formel II 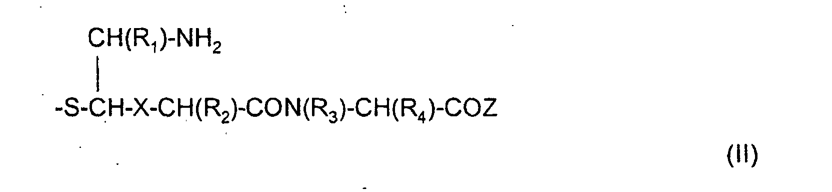 darstellt, das der symmetrischen Dischwefeleinheit des Inhibitors entspricht, wobei R₁, R₂, R₃, R₄, X und Z wie vorstehend definiert sind,
ihre pharmakologisch akzeptablen Additionssalze, die mit organischen oder anorganischen Säuren erhalten werden,
und Dimere von Verbindungen der Formel (I), die aus zwei Molekülen der Verbindung der Formel (I) bestehen, die identisch oder verschieden voneinander sein können und über ihre entsprechenden Schwefelatomen untereinander gekoppelt sind, wobei R eine Gruppe der Formel (II) darstellt, wie sie vorstehend definiert ist.

2. Verbindung nach Anspruch 1, **dadurch gekennzeichnet, dass** R₄ eine Alkylkette, eine Aryl-, Arylalkyl-, Cycloalkyl-, (Cycloalkyl)-Alkyl-, (Heteroaryl)-Alkyl-, Heterocycloalkyl- oder (Heterocycloalkyl)-Alkylgruppe, darstellt, die wenigstens durch eine CONH₂-, SO₃H-, SO₂NH₂-, PO₃H₂- oder eine Tetrazolylgruppe substituiert ist, wobei die SO₃H- und PO₃H₂-Gruppen geschützt sein können und
R₄ mit R₃ eine heterocyclische Verbindung mit 5 oder 6 Ringgliedern bildet, die mit wenigstens einer CO₂H-, CONH₂-, SO₃H-, SO₂NH₂- oder PO₃H₂-Gruppe substituiert ist, wobei die CO₂H-, SO₃H- und PO₃H₂-Gruppen wie in Anspruch 1 beschrieben geschützt sein können.

3. Verbindung nach Anspruch 1 oder 2, **dadurch gekennzeichnet, dass** R₄ und R₅ zusammen eine heterocyclische Verbindung mit 5 oder 6 Ringgliedem bilden, und die wenigstens mit einer CO₂H-, SONH₂-, SO₃H-, SO₂NH₂- oder PO₃H₂-Gruppe substituiert ist, wobei die CO₂H-, SO₃H-, und PO₃H₂-Gruppen wie in Anspruch 1 beschrieben geschützt sein können.

4. Verbindung nach einem der vorhergehenden Ansprüche, **dadurch gekennzeichnet, dass** X eine CONH-Funktion darstellt.

5. Verbindung nach einem der vorhergehenden Ansprüche, **dadurch gekennzeichnet, dass** R₂ eine Alkyl- oder Arylalkylkette darstellt, die ggf. substituiert ist.

6. Verbindung nach einem der Ansprüche 1 bis 5, **dadurch gekennzeichnet, dass** sie ausgewählt ist aus:
den N-[[(2S, 3R) und (2R, 3R), 3-Amino-, 2-Sulfhydryl-, 5-Sulfonat]pentanoyl] - L. Tyr-L.Sal-OH und
den N-[[(2S, 3R) und (2R, 3R), 3-Amino-, 2-Sulfhydryl-, 5-Sulfonat]pentanoyl] - L. Tyr-L. hSal-OH.

7. Verfahren zur Herstellung einer Verbindung mit der allgemeinen Formel I 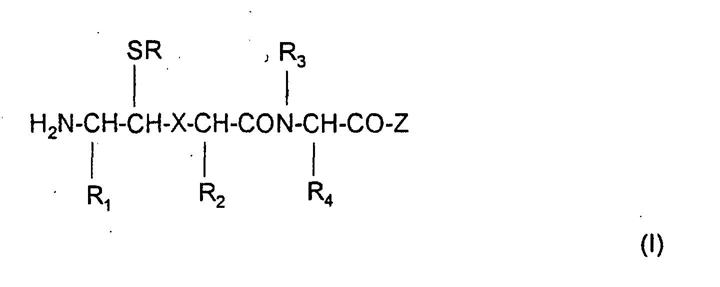 wobei R₁, R₂, R₃, R₄, R und Z wie in Anspruch 1 oder 2 definiert sind und X eine CONH-Gruppe darstellt, **dadurch gekennzeichnet, dass** er wenigstens die Kopplung eines Dipeptidesters der allgemeinen Formel III 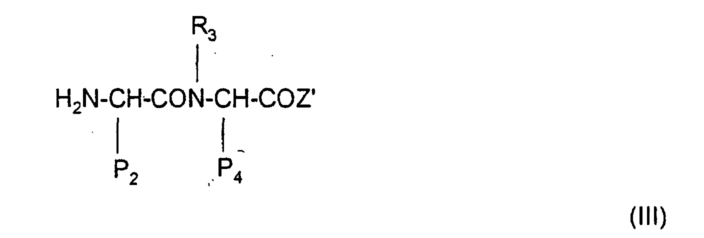 wobei:
- P₂ und P₄ den geschützten Formen von R₂ und R₄ entsprechen,
- Z' eine OC(CH₃)₃-, OCH₂-C₆H₅- oder NR"R"'-Gruppe darstellt, wobei R" und R''' unabhängig voneinander ein Wasserstoffatom oder eine Alkyl-, Aryl- oder Arylalkylgruppe darstellen, und wobei R" und R''' zusammen mit dem Stickstoffatom einen Heterocyclus mit 5 oder 6 Ringgliedern bilden, der ggf. ein zweites Heteroatom aufweist, ausgewählt aus O, S und N,
mit einer Verbindung der allgemeinen Formel IV 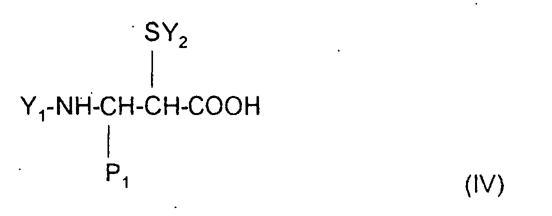 wobei:
- Y₁ eine Schutzgruppe darstellt,
- Y₂ eine Schutzgruppe darstellt,
- P₁ eine geschützte Form von R¹ darstellt,
unter ausreichenden Bedingungen, um über Verbindung V 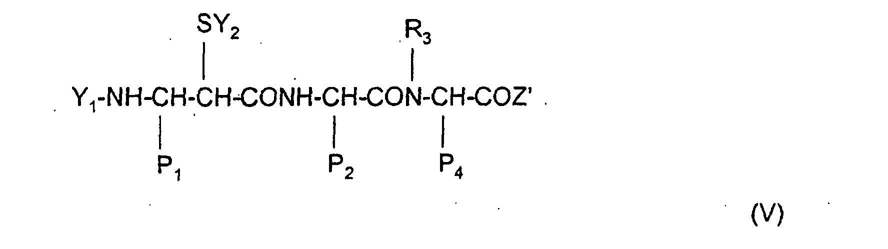 und ihrer Entschützung zur Verbindung der allgemeinen Formel I zu führen, wobei die Kupplungsreaktion in einem organischen Lösungsmittel in Gegenwart eines Kopplungsmittels und eines tertiären Amins bei einer Temperatur in der Größenordnung von 20°C durchgeführt wird.

8. Verfahren zur Herstellung einer Verbindung der allgemeinen Formel I 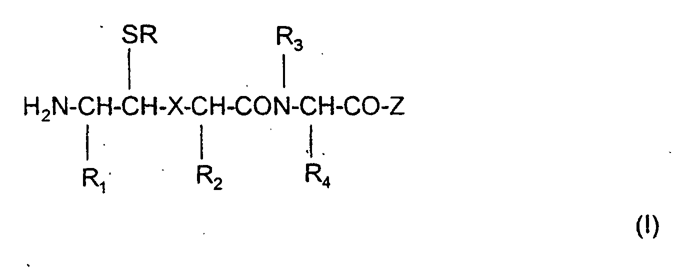 wobei R₁, R₂, R₃, R₄, R und Z wie in Anspruch 1 definiert sind und X eine Gruppe CH₂-NH darstellt, **dadurch gekennzeichnet, dass** das Verfahren wenigstens umfasst:
- die Kondensation einer Verbindung der allgemeinen Formel III 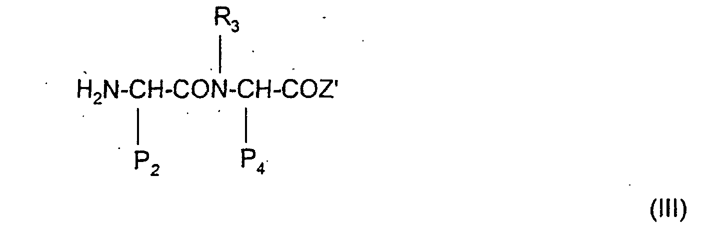 umfasst, wobei
- Z' eine OC(CH₃)₃-, OCH₂-C₆H₅- oder NR"R"'-Gruppe darstellt, wobei R" und R''' unabhängig voneinander ein Wasserstoffatom oder eine Alkyl-, Aryl- oder Arylalkylgruppe darstellt, und wobei R" und R''' zusammen mit dem Stickstoffatom einen Heterocyclus mit 5 oder 6 Ringgliedern bilden, der ggf. ein zweites Heteroatom aufweist, ausgewählt aus O, S und N,
mit einer Verbindung der allgemeinen Formel VI 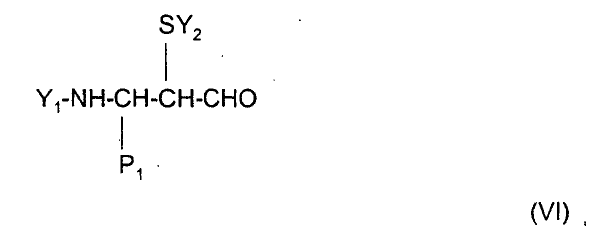 wobei Y₁, Y₂ und P₁ wie in Anspruch 6 definiert sind,
- die Reduktion des so gebildeten Zwischenprodukts um über die Verbindung der allgemeinen Formel VII 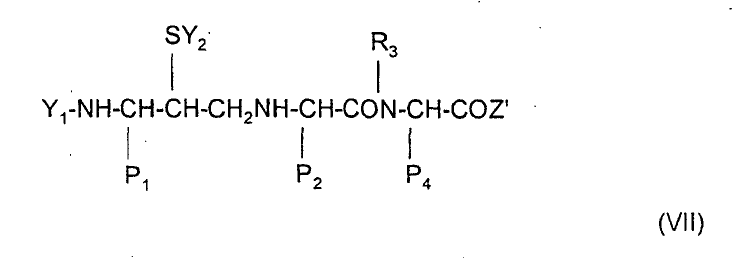 und ihrer Entschützung zur Verbindung der allgemeinen Formel I zu führen.

9. Verfahren nach einem der Ansprüche 7 oder 8, **dadurch gekennzeichnet, dass** die zwei asymmetrischen Kohlenstoffatome des Dipeptidesters der allgemeinen Formel III eine S-Konfiguration aufweisen.

10. Verwendung einer Verbindung nach einem der Ansprüche 1 bis 6 als selektiven Inhibitor für Aminopeptidase A.

11. Verbindung nach einem der Ansprüche 1 bis 6 zur Herstellung eines Medikaments zur Verminderung der Nahrungsaufnahme, zur Beeinflussung von Angstzuständen oder Panikzuständen oder zur Behandlung des essentiellen und sekundären erhöhten Bluthochdrucks, Herzschwäche und Nierenschwäche, Homöostasie, Myocarditis, oder Proteinurie bei Diabetikern.

12. Pharmazeutische Zusammensetzung umfassend als aktiven Wirkstoff wenigstens eine Verbindung nach einem der Ansprüche 1 bis 6.

13. Diagnosesystem zum Nachweis und zur Dosierung der Aminopeptidase A, **dadurch gekennzeichnet, dass** es wenigstens eine Verbindung nach den Ansprüchen 1 bis 6 umfasst.

14. Verbindung, ausgewählt aus den N-[[(2S, 3R) und (2R, 3R), 3-Amino, 5-Carboxy, 2-Sulfhydryl]pentanoyl] - L.Ile-L.(3R) (3-COOH) Pro-OH.

15. Verwendung einer Verbindung nach Anspruch 14 als selektiven Inhibitor für Aminopeptidase A.

16. Verwendung einer Verbindung nach Anspruch 14 zur Herstellung eines Medikaments zur Verminderung der Nahrungsaufnahme, zur Beeinflussung von Angst- oder Panikzuständen oder zur Behandlung des essentiellen und sekundären erhöhten Bluthochdrucks, Herzschwäche und Nierenschwäche, Homöostasie, Myocarditis, oder Proteinurie bei Diabetikern.

17. Verbindung, ausgewählt aus N-[[(2S; 3R) und (2R, 3R), 3-Amino, 5-Phosphonat, 2-Sulfhydryl]pentanoyl] -L.Ile -L.Glu-OH und dem N-[[(2S, 3R) und (2R, 3R), 3-Amino, 2-Sulfhydryl, 5-Sulfonat]pentanoyl]-L.Ile -L.Glu-NH₂.

18. Verwendung einer Verbindung nach Anspruch 17 als selektiven Inhibitor für Aminopeptidase.

19. Verwendung einer Verbindung nach Anspruch 17 zur Herstellung eines Medikaments zur Verminderung der Nahrungsaufnahme, zur Beeinflussung von Angst- oder Panikzuständen oder zur Behandlung des essentiellen und sekundären erhöhten Bluthochdrucks, Herzschwäche und Nierenschwäche, Homöostasie, Myocarditis, oder Proteinurie bei Diabetikern.

20. Verbindung nach einem der Ansprüche 1 bis 5, wobei die Pentylgruppe, die die SO₃H-Gruppe schützt, eine Neopentylgruppe ist.

## Claims

1. Compound with general formula I: 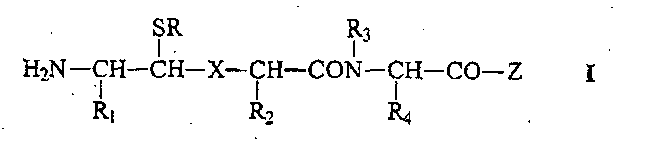 in which:
- R₁ represents alkyl, alkenyl or alkynyl chain, or a cycloalkyl or (cycloalkyl)alkyl group substituted by at least one group
- -COOH, possibly esterified by an alkyl group comprising from 2 to 12 carbon atoms,
- -SO₃H, possibly protected by a pentylic group,
- -PO₃H₂ possibly substituted by a group (-CH₂CH₂SCOR₅) with R₅ representing an alkyl group as C₁-C₄, a phenyl or benzyl group, or
- tetrazolyl.
- R₂ represents an alkyl chain or an aryl, arylalkyl, cycloalkyl, (cycloalkyl)alkyl, (heteroaryl)alkyl group, substituted or not by at least one group OH, OR, SR', NH₂, NHR', guanidinyl, COOH, CONH₂, of a halogen atom chosen from F, Cl, Br or I with R' representing an alkyl as C₁ to C₄, linear or branched.
- R₃ represents a hydrogen atom or a methyl group,
- R₄ represents
- an alkyl chain, an aryl, arylalkyl, cycloalkyl, (cycloalkyl)alkyl, (heteroaryl)alkyl, heterocycloalkyl or (heterocycloalkyl)alkyl group substituted by at least one CONH₂, SO₃H, SO₂NH₂, PO₃H₂ or tetrazolyl group, with the SO₃H group being protected by a pentylic group and the PO₃H₂ group being protected by a (-CH₂CH₂SCOR₅) group with R₅ representing an alkyl group as C₁-C₄, a phenyl or benzyl group,
- an aryl, arylalkyl, cycloalkyl, (cycloalkyl)alkyl, (heteroaryl)alkyl, heterocycloalkyl or (heterocycloalkyl)alkyl group substituted by at least one CO₂H group, which can be protected as described above, i.e. possibly esterified by an alkyl group containing from 2 to 12 carbon atoms, or
- R₃ and R₄ can together make a heterocyclic compound, with 5 or 6 links, substituted by at least one CO₂H, CONH₂, SO₃H, SO₂NH₂ or PO₃H₂ with the groups CO₂H, SO₃H and PO₃H₂, where necessary protected as described above,
- X represents a CONH or CH₂NH group,
- Z represents an OH, OCH₂-C₆H₅ or NR"R''' group in which R" and R''' can represent, independently of each other, a hydrogen atom or an alkyl, aryl or arylalkyl group, with R" and R''' being able to constitute together, with ;the nitrogen atom, a heterocycle with 5 or 6 links possessing where necessary a 2^{nd} hetero-atom chosen from O, S and N, and
- R represents a hydrogen atom or a formula II group 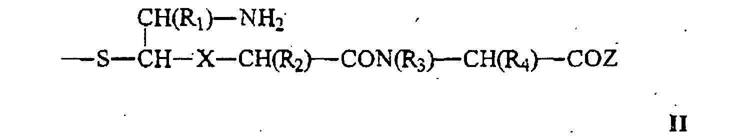 corresponding with symmetrical disulphide of the inhibitor with R₁, R₂, R₃, R₄, X and Z being as defined above,
their additive salts obtained with pharmacologically acceptable mineral or organic acids, and the dimers of compounds in formula (I), consisting of two molecules of compounds in formula (I), identical or different, coupled with each other at the level of their respective sulphur atoms, R then representing a group in formula (II) as defined above.

2. Compound according to Claim 1, **characterised in that**
- R₄ represents an alkyl chain, an aryl, arylalkyl, cycloalkyl, (cycloalkyl)alkyl, (heteroaryl)alkyl, heterocycloalkyl or (heterocyclo-alkyl)alkyl group substituted by at least a CONH₂, SO₃H, SO₂NH₂, PO₃H₂ group or tetrazolyl, with the SO₃H and PO₃H₂ groups being able to be protected, or
- R₄ constitutes with R₃ a heterocyclic compound with 5 or 6 links, substituted by at least one CO₂H, CONH₂, SO₃H, SO₂NH₂ or PO₃H₂ group with the groups CO₂H, SO₃H and PO₃H₂ being able to be protected as described in Claim 1.

3. Compound according to Claim 1 or 2, **characterised in that** R₄ and R₃ together constitute a heterocyclic compound with 5 or 6 links, substituted by at least one CO₂H, CONH₂, SO₃H, SO₂NH₂ or PO₃H₂ group with the groups CO₂H, SO₃H and PO₃H₂ being able to be protected as described in Claim 1.

4. Compound according to one of the above claims, **characterised in that** X represents a CONH function.

5. Compound according to one of the above claims, **characterised in that** R₂ represents an alkyl or arylalkyl chain substituted as necessary.

6. Compound according to at least one of the Claims 1 to 5, **characterised in that** it is chosen from among:
the N-[[(2S,3R) and (2R,3R), 3-amino, 2-sulph-hydryl, 5-sulphonate] pentanoyl]-L.Tyr-L.Sal-OH; and
the N-[[(2S,3R) and (2R,3R), 3-amino, 2-sulph-hydryl, 5-sulphonate] pentanoyl]-L.Tyr-L.hSal-OH.

7. Process for preparing a compound with the general formula I 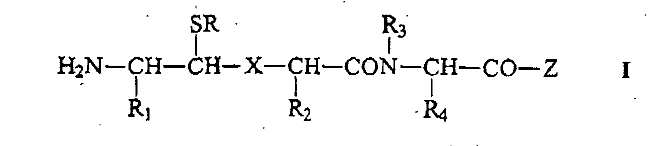 with R₁, R₂, R₃, R₄, R and Z as defined in Claim 1 or 2 and X representing a CONH group, **characterised in that** it comprises at least the coupling of a dipeptide ester of general formula III 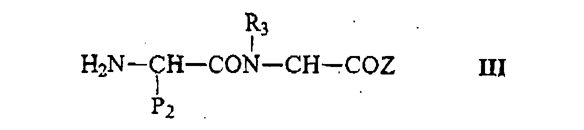 in which
- P₂ and P₄ correspond to protected forms of R₂ and R₄
- Z' represents a group OC(CH₃)₃, OCH₂-C₆H₅ or NR"R''' in which R" and R''' can represent, independently of each other, a hydrogen atom or an alkyl, aryl or arylalkyl group, with R" and R''' being able to constitute together, with the nitrogen atom, a heterocycle with 5 or 6 links possessing where necessary a 2^{nd} hetero-atom chosen from among O, S and N, with a compound with the general formula IV 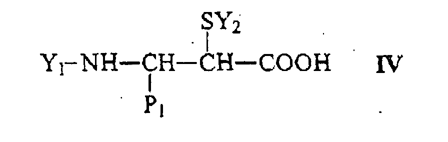
in which
- Y₁ represents a protective group
- Y₂ represents a protective group
- P₁ represents a protected form of R₁
under conditions sufficient to lead via compound V 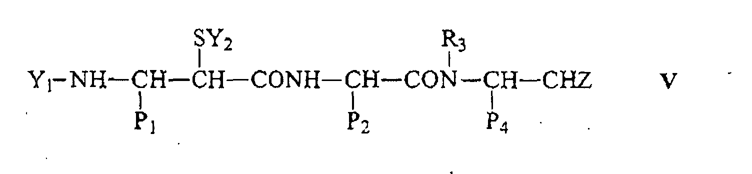 and its deprotection to the said compound in general formula I, the coupling reaction being made in an organic solvent, in the presence of a coupling agent and a tertiary amine and at a temperature in the region of 20°C

8. Process for preparing a compound with the general formula I 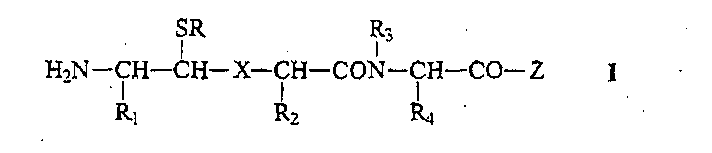 in which R_{1,} R_{2,} R₃, R₄, R and Z are as defined in Claim 1 and X represents a CH₂-NH group, **characterised in that** it includes at least:
- the condensation of a compound of the general formula III 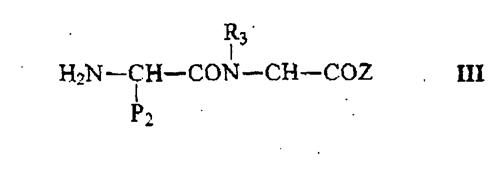
- Z' represents a group OC(CH₃)₃, OCH₂-C₆H₅ or NR"R''' in which R" and R''' can represent, independently of each other, a hydrogen atom or an alkyl, aryl or arylalkyl group, with R" and R''' being able to constitute together, with the nitrogen atom, a heterocycle with 5 or 6 links possessing where necessary a 2^{nd} hetero-atom chosen from O, S and N, with a compound of general formula VI 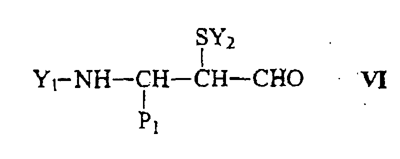
in which Y₁, Y₂ and P₁ are as defined in Claim 6,
- the reduction of the intermediary thus formed to lead via the general formula VII compound 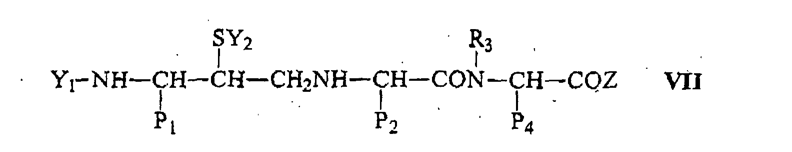 and its deprotection to the said compound in general formula I.

9. Process according to one of the Claims 7 or 8, **characterised in that** the two asymmetrical carbons of the dipeptide ester of general formula III have an S configuration.

10. Use of a compound as defined according to one of the Claims 1 to 6 as a selective inhibitor with regard to amino peptidase A.

11. Use of a compound as defined according to one of the Claims I to 6 to prepare a medicament intended to reduce the alimentary intake, modulate states of anxiety or panic attacks or treat essential and secondary arterial high blood pressure, cardiac and renal deficiency, hydrodynamic homeostasis problems, myocardial infarction or proteinuria in people with diabetes.

12. Pharmaceutical composition comprising as the active principle at least one compound according to one of the Claims 1 to 6.

13. Diagnostic system to detect and dose amino peptidase A, **characterised in that** it includes at least one compound as defined in one of the Claims 1 to 6.

14. Compound chosen from among the N-[[(2S,3R) and (2R,3R), 3-amino, 5-carboxy, 2-sulph-hydryl] pentanoyl]-L.Ile-L.(3R)(3-COOH)Pro-OH,

15. Use of a compound as defined according to Claim 14, as a selective inhibitor with regard to amino peptidase A.

16. Use of a compound as defined according to Claim 14 to prepare a medicament intended to reduce the alimentary intake, modulate states of anxiety or panic attacks or treat essential and secondary arterial high blood pressure, cardiac and renal deficiency. hydrodynamic homeostasis, myocardial infarction or proteinuria in people with diabetes.

17. Compound chosen from among the N-[[(2S,3R) and (2R,3R), 3-amino, 5-phosphnate, 2-sulph-hydryl] pentanoyl]-L.Ile-L.Glu -OH and the N-[[(2S,3R) and (2R,3R), 3-amino, 2-sulph-hydryl, 5-sulphonate] pentanoyl]-L.Ile-L.Glu-NH_{2.}

18. Use of a compound as defined in Claim 17, as a selective inhibitor with regard to amino peptidase.

19. Use of a compound as defined according to Claim 17, to prepare a medicament intended to reduce the alimentary intake, modulate states of anxiety or panic attacks or treat essential and secondary arterial high blood pressure, cardiac and renal deficiency, hydrodynamic homeostasis problems, myocardial infarction or proteinuria in people with diabetes.

20. Compound according to any one of the Claims 1 to 5, in which the pentylic group protecting SO₃H is a neopentyl.
